# EUROPEAN PATENT SPECIFICATION

(11) **EP 1 521 823 B1**
(45) Date of publication and mention of the grant of the patent: **23.05.2012**
(21) Application number: 03763879.8
(22) Date of filing: 16.07.2003
(51) Int. Cl.: C12N 9/02

(54) **NEURONALLY EXPRESSED TRYPTOPHANE HYDROXYLASE AND ITS USE**
NEURONAL EXPRIMIERTE TRYPTOPHANHYDROXYLASE UND DEREN VERWENDUNG
TRYPTOPHANE HYDROXYLASE EXPRIMEE DE MANIERE NEURONALE ET SON UTILISATION

(30) Priority: 16.07.2002 DE 10232151
(43) Date of publication of application: 13.04.2005
(73) Proprietor: Max Delbrück Centrum für Molekulare Medizin (MDC) Berlin-Buch;, 13125 Berlin (DE)
(72) Inventor: WALTHER, Diego, J., 14195 Berlin (DE); BADER, Michael, 13125 Berlin (DE)
(74) Representative: Krauss, Jan
(86) International application number: PCT/EP2003/007744
(87) International publication number: WO 2004/007704

(56) References cited:
- WO-A-02/17891
- WO-A-02/097039
- CHUNG Y I ET AL: "Immunochemical characterization of brain and pineal tryptophan hydroxylase" JOURNAL OF KOREAN MEDICAL SCIENCE, vol. 16, no. 4, August 2001 (2001-08), pages 489-497, XP009019879 ISSN: 1011-8934
- WALTHER D J ET AL: "Synthesis of serotonin by a second tryptophan hydroxylase isoform." SCIENCE (WASHINGTON D C), vol. 299, no. 5603, 3 January 2003 (2003-01-03), page 76, XP002259156 ISSN: 0036-8075 (ISSN print)
- CASH C D: "Why tryptophan hydroxylase is difficult to purify: A reactive oxygen-derived species-mediated phenomenon that may be implicated in human pathology" GENERAL PHARMACOLOGY, vol. 30, no. 4, April 1998 (1998-04), pages 569-574, XP002259157 ISSN: 0306-3623
- WALTHER D J ET AL: "A unique central tryptophan hydroxylase isoform" BIOCHEMICAL PHARMACOLOGY, vol. 66, no. 9, 1 November 2003 (2003-11-01), pages 1673-1680, XP002259158

### SEQUENCE LISTING

<110> Max-Delbrück-Centrum für Molekulare Medizin
<120> Neuronally expressed tryptophane hydroxylase and its use
<130> M30317PCT
<160> 14
<170> PatentIn version 3.2
<210> 1
   <211> 2350
   <212> DNA
   <213> homo sapiens
<400> 1
   cattgctctt cagcaccagg gttctggaca gcgccccaag caggcagctg atcgcacgcc      60
   ccttcctctc aatctccgcc agcgctgcta ctgcccctct agtaccccct gctgcagaga     120
   aagaatatta caccgggatc catgcagcca gcaatgatga tgttttccag taaatactgg     180
   gcacggagag ggttttccct ggattcagca gtgcccgaag agcatcagct acttggcagc     240
   tcaacactaa ataaacctaa ctctggcaaa aatgacgaca aaggcaacaa gggaagcagc     300
   aaacgtgaag ctgctaccga aagtggcaag acagcagttg ttttctcctt gaagaatgaa     360
   gttggtggat tggtaaaagc actgaggctc tttcaggaaa aacgtgtcaa catggttcat     420
   attgaatcca ggaaatctcg gcgaagaagt tctgaggttg aaatctttgt ggactgtgag     480
   tgtgggaaaa cagaattcaa tgagctcatt cagttgctga aatttcaaac cactattgtg     540
   acgctgaatc ctccagagaa catttggaca gaggaagaag agctagagga tgtgccctgg     600
   ttccctcgga agatctctga gttagacaaa tgctctcaca gagttctcat gtatggttct     660
   gagcttgatg ctgaccaccc aggatttaag gacaatgtct atcgacagag aagaaagtat     720
   tttgtggatg tggccatggg ttataaatat ggtcagccca ttcccagggt ggagtatact     780
   gaagaagaaa ctaaaacttg gggtgttgta ttccgggagc tctccaaact ctatcccact     840
   catgcttgcc gagagtattt gaaaaacttc cctctgctga ctaaatactg tggctacaga     900
   gaggacaatg tgcctcaact cgaagatgtc tccatgtttc tgaaagaaag gtctggcttc     960
   acggtgaggc cggtggctgg atacctgagc ccacgagact ttctggcagg actggcctac    1020
   agagtgttcc actgtaccca gtacatccgg catggctcag atcccctcta caccccagaa    1080
   ccagacacat gccatgaact cttgggacat gttccactac ttgcggatcc taagtttgct    1140
   cagttttcac aagaaatagg tctggcgtct ctgggagcat cagatgaaga tgttcagaaa    1200
   ctagccacgt gctatttctt cacaatcgag tttggccttt gcaagcaaga agggcaactg    1260
   cgggcatatg gagcaggact cctttcctcc attggagaat taaagcacgc cctttctgac    1320
   aaggcatgtg tgaaagcctt tgacccaaag acaacttgct tacaggaatg ccttatcacc    1380
   accttccagg aagcctactt tgtttcagaa agttttgaag aagccaaaga aaagatgagg 1440
   gactttgcaa agtcaattac ccgtcccttc tcagtatact tcaatcccta cacacagagt 1500
   attgaaattc tgaaagacac cagaagtatt gaaaatgtgg tgcaggacct tcgcagcgac 1560
   ttgaatacag tgtgtgatgc tttaaacaaa atgaaccaat atctggggat ttgatgcctg 1620
   gaactatgtt gttgccagca tgatcttttt ggggcttagc agcagttcag tcaatgtcat 1680
   ataacgcaaa taaccttctg tgtcatggct tggctaataa gcatgcaatt ccatatatct 1740
   ataccatctt gtaactcact gtgttagtat ataaagcacc ataagaaatc caatggcaga 1800
   taacctgaaa taacgtatta tgtttaaaca tcttaaaaag atttgacatt cctgcttagt 1860
   gtccttaacc aaactgcatc tagttaaaat ttgtaacaaa tagccctctt atgagtctca 1920
   tttatgccct tttctttttc agatctaagc ctttcctctg tgttcattag ataaaatgaa 1980
   aaaaagcagt gaagctgttt ccattttcaa tagtatcagt gttttcacgc attatttgag 2040
   ataaacccag aattgtagga aacttcccat cacaataaca aaggttcaat attctatttc 2100
   aaaaattgtt gaggtaacac agcagttgga atgattttta ggttgagtat ttacacaatg 2160
   caagaaaaca cctttttaca aatggaatta tgtaggttgc gttgaccttg tagaacctga 2220
   gttatgacaa gcttcctgaa gtattttgga agatagtact tccggaaagg acattaggaa 2280
   agactaaaca gtggacaatc aatcttggga ctatgaattt tatgctggaa taaagtaaat 2340
   tatcatgttc 2350
<210> 2
   <211> 490
   <212> PRT
   <213> homo sapiens
<400> 2
   Met Gln Pro Ala Met Met Met Phe Ser Ser Lys Tyr Trp Ala Arg Arg
   1 5 10 15
   Gly Phe Ser Leu Asp Ser Ala Val Pro Glu Glu His Gln Leu Leu Gly
   20 25 30
   Ser Ser Thr Leu Asn Lys Pro Asn Ser Gly Lys Asn Asp Asp Lys Gly
   35 40 45
   Asn Lys Gly Ser Ser Lys Arg Glu Ala Ala Thr Glu Ser Gly Lys Thr
   50 55 60
   Ala Val Val Phe Ser Leu Lys Asn Glu Val Gly Gly Leu Val Lys Ala
   65 70 75 80
   Leu Arg Leu Phe Gln Glu Lys Arg Val Asn Met Val His Ile Glu Ser
   85 90 95
   Arg Lys Ser Arg Arg Arg Ser Ser Glu Val Glu Ile Phe Val Asp Cys
   100 105 110
   Glu Cys Gly Lys Thr Glu Phe Asn Glu Leu Ile Gln Leu Leu Lys Phe
   115 120 125
   Gln Thr Thr Ile Val Thr Leu Asn Pro Pro Glu Asn Ile Trp Thr Glu
   130 135 140
   Glu Glu Glu Leu Glu Asp Val Pro Trp Phe Pro Arg Lys Ile Ser Glu
   145 150 155 160
   Leu Asp Lys Cys Ser His Arg Val Leu Met Tyr Gly Ser Glu Leu Asp
   165 170 175
   Ala Asp His Pro Gly Phe Lys Asp Asn Val Tyr Arg Gln Arg Arg Lys
   180 185 190
   Tyr Phe Val Asp Val Ala Met Gly Tyr Lys Tyr Gly Gln Pro Ile Pro
   195 200 205
   Arg Val Glu Tyr Thr Glu Glu Glu Thr Lys Thr Trp Gly Val Val Phe
   210 215 220
   Arg Glu Leu Ser Lys Leu Tyr Pro Thr His Ala Cys Arg Glu Tyr Leu
   225 230 235 240
   Lys Asn Phe Pro Leu Leu Thr Lys Tyr Cys Gly Tyr Arg Glu Asp Asn
   245 250 255
   Val Pro Gln Leu Glu Asp Val Ser Met Phe Leu Lys Glu Arg Ser Gly
   260 265 270
   Phe Thr Val Arg Pro Val Ala Gly Tyr Leu Ser Pro Arg Asp Phe Leu
   275 280 285
   Ala Gly Leu Ala Tyr Arg Val Phe His Cys Thr Gln Tyr Ile Arg His
   290 295 300
   Gly Ser Asp Pro Leu Tyr Thr Pro Glu Pro Asp Thr Cys His Glu Leu
   305 310 315 320
   Leu Gly His Val Pro Leu Leu Ala Asp Pro Lys Phe Ala Gln Phe Ser
   325 330 335
   Gln Glu Ile Gly Leu Ala Ser Leu Gly Ala Ser Asp Glu Asp Val Gln
   340 345 350
   Lys Leu Ala Thr Cys Tyr Phe Phe Thr Ile Glu Phe Gly Leu Cys Lys
   355 360 365
   Gln Glu Gly Gln Leu Arg Ala Tyr Gly Ala Gly Leu Leu Ser Ser Ile
   370 375 380
   Gly Glu Leu Lys His Ala Leu Ser Asp Lys Ala Cys Val Lys Ala Phe
   385 390 395 400
   Asp Pro Lys Thr Thr Cys Leu Gln Glu Cys Leu Ile Thr Thr Phe Gln
   405 410 415
   Glu Ala Tyr Phe Val Ser Glu Ser Phe Glu Glu Ala Lys Glu Lys Met
   420 425 430
   Arg Asp Phe Ala Lys Ser Ile Thr Arg Pro Phe Ser Val Tyr Phe Asn
   435 440 445
   Pro Tyr Thr Gln Ser Ile Glu Ile Leu Lys Asp Thr Arg Ser Ile Glu
   450 455 460
   Asn Val Val Gln Asp Leu Arg Ser Asp Leu Asn Thr Val Cys Asp Ala
   465 470 475 480
   Leu Asn Lys Met Asn Gln Tyr Leu Gly Ile
   485 490
<210> 3
   <211> 2638
   <212> DNA
   <213> mus musculus
<400> 3
   cactgctctt cagcaccagg gttctggaca gcgccccgag caggcagctg ccactgcagt 60
   tcctccttca tctctgccaa ggccgcccct ctggtccccc ctgctgctga gaaagaaaat 120
   tacatcggga gccatgcagc ccgcaatgat gatgttttcc agtaaatact gggccaggag 180
   agggttgtcc ttggattctg ctgtgccaga agatcatcag ctacttggca gcttaacaca 240
   aaataaggct atcaaaagcg aggacaagaa aagcggcaaa gagcccggca aaggcgacac 300
   cacagagagc agcaagacag cagttgtgtt ctccttgaag aatgaagttg gtgggctggt 360
   gaaagcactt agactattcc aggaaaaaca tgtcaacatg cttcatatcg aatccaggcg 420
   gtcccggcga agaagttctg aagtcgaaat cttcgtggac tgcgaatgtg gcaaaacgga 480
   attcaatgag ctcatccagt tgctgaaatt tcagaccacc attgtgaccc tgaatccgcc 540
   tgagagcatt tggacggagg aagaagatct cgaggatgtg ccgtggttcc ctcggaagat 600
   ctctgagtta gacagatgct ctcaccgagt cctcatgtac ggcaccgagc ttgatgccga 660
   ccatccagga tttaaggaca atgtctatcg acagaggagg aagtattttg tggatgtggc 720
   catgggctat aaatatggtc agcccattcc cagggtcgag tacacagaag aagagactaa 780
   aacttggggt gttgtgttcc gggagctctc caaactctac ccgactcatg cttgccggga 840
   gtacctgaaa aacctccccc tgctgaccaa gtactgtggc tacagggaag acaacgtgcc 900
   gcaactggaa gacgtctcca tgtttctgaa agagcgatct ggcttcacag tgagaccagt 960
   ggctggctac ctgagcccaa gagacttcct ggcgggcctg gcctacagag tattccactg 1020
   cacccagtac gtgcggcatg gctccgaccc cctctacacc ccggaaccag atacatgcca 1080
   tgaactcttg ggacacgtgc cactgcttgc ggatcccaag tttgctcagt tttcccaaga 1140
   gataggctta gcgtctctgg gagcctcaga tgaggacgtt cagaaactag ccacgtgcta 1200
   tttcttcaca atcgagttcg gcctttgcaa gcaagagggt caactgcggg cgtatggagc 1260
   agggttactt tcgtccatcg gagaattgaa gcatgctctt tccgacaagg cgtgtgtgaa 1320
   atcctttgac ccaaagacga cctgcttgca ggaatgccta atcaccacct ttcaggacgc 1380
   ttactttgtt tcggacagtt ttgaagaagc caaagaaaag atgagggact ttgcaaagtc 1440
   aattacccgt cccttctcgg tatacttcaa ccgctacacg cagagcattg aaattctgaa 1500
   agacaccaga agtattgaga atgtggtgca ggacctgcgc agtgatttga acacagtgtg 1560
   tgatgccttg aataaaatga accaatatct ggggatttga tgcctagaac cagagttatt 1620
   gtcagcatga gctcttgggg ggtgtagcaa caatgcagtc aatgttatcc aacatcaaca 1680
   actttctgtg tcatggttgg ctagtaagca tgcaattctg tatgtccata cctctgtgta 1740
   acttaataac acaaaaatgc tctaaagaac ccatgcagat aaccactcac catttgaaag 1800
   attgtgatcc tatttggaca tctcaagtag agttgacatt tctgattagc gaacaaactg 1860
   ttaacttaag caaactgtga ctttgaaatc tgtagcaaac attcctcgca caattccagt 1920
   cggtgagttg tggaactttt cttccttgga cctgagactt tcctctgtgt tcattagata 1980
   aaatgaaaat agttgggagg tggtttctat tttcaatagt atccgtgtta tttgagataa 2040
   actagagttg ctccacgctt tgcatcacag caacaaagga tttaatattc tacttcagaa 2100
   gctgttcaga aacacagcag ttgggatgga tgtagactga gtgttcagac aatgcaagca 2160
   aagaaaagtt ttgataaaca ggatatatag gttgtactga cctcgttgaa accaatttgt 2220
   ggcaagcttc ctgaagagct tctggaagga aacacttgaa caaagaatat tcgggaagct 2280
   taaacagaag ggatgaaaat cttggaactg tgaatgtatt gttaggatag agtgaattat 2340
   cactgcaggc ttttgactcc ttttgcttag actgagaacc tcaaatccca cagggatgta 2400
   aataccatct ctgattccaa agagttggag acggagtcgt agagaaacaa agggatttgc 2460
   ttcagttagg tctgatgaga tgtgccatgg tcataagcca ctgccctttt atgttggaca 2520
   tctgacaagt ctactgtagt gtacatgcat gtttatgtat tgacacagaa agaaaattat 2580
   tgcttataaa atgaatgctt ctcaataaac agaatcttgc ccccaaaaaa aaaaaaaa 2638
<210> 4
   <211> 488
   <212> PRT
   <213> mus musculus
<400> 4
   Met Gln Pro Ala Met Met Met Phe Ser Ser Lys Tyr Trp Ala Arg Arg
   1               5                   10                  15
   Gly Leu Ser Leu Asp Ser Ala Val Pro Glu Asp His Gln Leu Leu Gly
               20                  25                  30
   Ser Leu Thr Gln Asn Lys Ala Ile Lys Ser Glu Asp Lys Lys Ser Gly
           35                  40                  45
   Lys Glu Pro Gly Lys Gly Asp Thr Thr Glu Ser Ser Lys Thr Ala Val
       50                  55                  60
   Val Phe Ser Leu Lys Asn Glu Val Gly Gly Leu Val Lys Ala Leu Arg
   65                  70                  75                  80
   Leu Phe Gln Glu Lys His Val Asn Met Leu His Ile Glu Ser Arg Arg
                   85                  90                  95
   Ser Arg Arg Arg Ser Ser Glu Val Glu Ile Phe Val Asp Cys Glu Cys
               100                 105                 110
   Gly Lys Thr Glu Phe Asn Glu Leu Ile Gln Leu Leu Lys Phe Gln Thr
           115                 120                 125
   Thr Ile Val Thr Leu Asn Pro Pro Glu Ser Ile Trp Thr Glu Glu Glu
       130                 135                 140
   Asp Leu Glu Asp Val Pro Trp Phe Pro Arg Lys Ile Ser Glu Leu Asp
   145                 150                 155                 160
   Arg Cys Ser His Arg Val Leu Met Tyr Gly Thr Glu Leu Asp Ala Asp
                   165                 170                 175
   His Pro Gly Phe Lys Asp Asn Val Tyr Arg Gln Arg Arg Lys Tyr Phe
               180                 185                 190
   Val Asp Val Ala Met Gly Tyr Lys Tyr Gly Gln Pro Ile Pro Arg Val
           195                 200                 205
   Glu Tyr Thr Glu Glu Glu Thr Lys Thr Trp Gly Val Val Phe Arg Glu
       210                 215                 220
   Leu Ser Lys Leu Tyr Pro Thr His Ala Cys Arg Glu Tyr Leu Lys Asn
   225                 230                 235                 240
   Leu Pro Leu Leu Thr Lys Tyr Cys Gly Tyr Arg Glu Asp Asn Val Pro
                   245                 250                 255
   Gln Leu Glu Asp Val Ser Met Phe Leu Lys Glu Arg Ser Gly Phe Thr
               260                 265                 270
   Val Arg Pro Val Ala Gly Tyr Leu Ser Pro Arg Asp Phe Leu Ala Gly
           275                 280                 285
   Leu Ala Tyr Arg Val Phe His Cys Thr Gln Tyr Val Arg His Gly Ser
       290                 295                 300
   Asp Pro Leu Tyr Thr Pro Glu Pro Asp Thr Cys His Glu Leu Leu Gly
   305                 310                 315                 320
   His Val Pro Leu Leu Ala Asp Pro Lys Phe Ala Gln Phe Ser Gln Glu
                   325                 330                 335
   Ile Gly Leu Ala Ser Leu Gly Ala Ser Asp Glu Asp Val Gln Lys Leu
               340                 345                 350
   Ala Thr Cys Tyr Phe Phe Thr Ile Glu Phe Gly Leu Cys Lys Gln Glu
           355                 360                 365
   Gly Gln Leu Arg Ala Tyr Gly Ala Gly Leu Leu Ser Ser Ile Gly Glu
       370                 375                 380
   Leu Lys His Ala Leu Ser Asp Lys Ala Cys Val Lys Ser Phe Asp Pro
   385                 390                 395                 400
   Lys Thr Thr Cys Leu Gln Glu Cys Leu Ile Thr Thr Phe Gln Asp Ala
                   405                 410                 415
   Tyr Phe Val Ser Asp Ser Phe Glu Glu Ala Lys Glu Lys Met Arg Asp
               420                 425                 430
   Phe Ala Lys Ser Ile Thr Arg Pro Phe Ser Val Tyr Phe Asn Arg Tyr
   435 440 445
   Thr Gln Ser Ile Glu Ile Leu Lys Asp Thr Arg Ser Ile Glu Asn Val
   450 455 460
   Val Gln Asp Leu Arg Ser Asp Leu Asn Thr Val Cys Asp Ala Leu Asn
   465 470 475 480
   Lys Met Asn Gln Tyr Leu Gly Ile
   485
<210> 5
   <211> 2581
   <212> DNA
   <213> rattus norvegicus
<400> 5
   cagggttctg gacagcgcct cgagcagcca gctgccgctc accttcctcc tacatctctg 60
   ccaaggctgc ccctctgatc ccccctgctg ctgagaaaga aaattacatc gggatccatg 120
   cagcccgcaa tgatgatgtt ttccagtaaa tactgggcca ggagagggtt gtccttggat 180
   tcagcggtgc cagaagagca tcagatactt ggcggcttaa cacaaaataa ggctaccgct 240
   agcaaaagcg aggacaagag aagcggcaaa gacacttcgg agagcagcaa gactgcggtt 300
   gtgttctccc tgaagaatga agttggcggg ctggtgagag cactgagact cttccaggaa 360
   aaacacgtca acatgctcca tattgaatcc aggaggtccc ggcgaagaag ttctgaagtc 420
   gaaatcttcg tggactgtga atgtggcaaa acagaattca acgagctcat tcagttgctc 480
   aagtttcaga ccaccattgt gacgctgaat ccacctgaca acatttggac ggaggaagaa 540
   gaactagagg atgtgccgtg gttccctcgg aagatctctg agttagacag atgctctcac 600
   agagtcctca tgtacggcac cgagcttgac gccgaccacc caggattcaa ggacaacgtc 660
   tatcgacaga ggaggaagta ttttgtggat gtggccatgg gttataaata tggccagccc 720
   attcccaggg tggaatacac agaagaagag actaaaactt ggggtgttgt gtttcgggag 780
   ctctccaaac tctaccccac tcatgcttgc cgagagtacc tgaaaaactt ccccctgctg 840
   accaagtact gcggctacag ggaagacaac gtcccgcagc tggaagacgt ctccatgttt 900
   ctgaaagagc gatctggctt cacagtgaga ccagtggctg gctacctgag cccaagagac 960
   ttcctggctg ggctggccta cagagtattc cactgcactc agtacgtgcg gcatggctcc 1020
   gaccccctct acaccccgga accagacaca tgccatgagc tcctgggaca tgtgccactg 1080
   ctggcggatc ccaagttcgc tcagttttct caagaaatag gcttagcctc tctgggagcc 1140
   tcagatgaag acgttcagaa actggccacg tgctatttct tcacaatcga gttcggcctt 1200
   tgcaagcaag aaggtcaact gcgggcgtac ggagcagggt tactttcctc catcggagaa 1260
   ttgaagcatg ctctttctga caaggcgtgt gtaaaagcct ttgacccgaa gacaacctgc 1320
   ttgcaggaat gcctaatcac caccttccaa gatgcttact ttgtttctga aagttttgaa 1380
   gaagccaaag agaagatgag ggattttgca aagtcaatta cccgtccttt ctcagtatat 1440
   ttcaacccct acacacagag cattgaaatt ctgaaagaca ccagaagtat tgagaatgtg 1500
   gtgcaggacc tgcgcagtga tttgaacacc gtgtgcgacg ccttgaataa aatgaaccaa 1560
   tatttgggga tttgagccta ttgtcagcac gagctcttgg gggcttagca acaatgcagt 1620
   caatgttatc caacatcaac aactttctgt gtcatggctg gctagtaagc atgcaattcc 1680
   atgtgtctat acctctatgt aacttaacat acaaaaatga tctaagaaac ccaggcagat 1740
   gaccattcag cattttaaag attgtgatct atttgaacat ctcaagtaga tttgacattt 1800
   ctgattagtg agcaaactgt aacttaagca aactgtgtct ttaaaatttg tagccaacat 1860
   tcctcacaca attccagctg ctgagtcctt gaccttttct tccttggacc tgagtctttc 1920
   ctctgtgttc attagataaa atgaaaacag ttgggaggtg gtttctactt tcaatagtat 1980
   tggtgttctc tgagataaac tagagttgct ccaagcttcg catcacagta acaaaagatt 2040
   taatatttta cttcagaagc tgttcagaaa cacagcgatt ggaatgaatc tggactgagt 2100
   gtttagacaa tgcaagaaaa gaaaaatttt gataaacagg atatatagat tgcactgacc 2160
   ttgttgaaac caatttgtgg tacgcttcct gaagtgcttt tggaaggaaa cactttgaca 2220
   aagaatattt ggaaaggtta aacagaaggg aagaaaatct tggaactgtg aatgtgtcat 2280
   tagaataaag tgaattatca gtgcaggtgt gactcctttc tcttacactg agaaccccaa 2340
   atcctgcagg gatgtgagta ccatctctga ttccgaagat ttggaaaccg agtcacagag 2400
   aaacaaaggg atttgcttca gttaggtctg ttggctgggg gtgcagtcat aatccccccc 2460
   cccctttatg ttggacttct ggcaagtcta ctgtagtgta catgcgggtt tatgtatgga 2520
   caaaaaaaga aaactaatgc gtataaaact aatgcttctc aataaacaga aacttgcccc 2580
   c 2581
<210> 6
   <211> 485
   <212> PRT
   <213> rattus norvegicus
<400> 6
   Met Gln Pro Ala Met Met Met Phe Ser Ser Lys Tyr Trp Ala Arg Arg
   1 5 10 15
   Gly Leu Ser Leu Asp Ser Ala Val Pro Glu Glu His Gln Ile Leu Gly
   20 25 30
   Gly Leu Thr Gln Asn Lys Ala Thr Ala Ser Lys Ser Glu Asp Lys Arg
   35 40 45
   Ser Gly Lys Asp Thr Ser Glu Ser Ser Lys Thr Ala Val Val Phe Ser
   50 55 60
   Leu Lys Asn Glu Val Gly Gly Leu Val Arg Ala Leu Arg Leu Phe Gln
   65 70 75 80
   Glu Lys His Val Asn Met Leu His Ile Glu Ser Arg Arg Ser Arg Arg
   85 90 95
   Arg Ser Ser Glu Val Glu Ile Phe Val Asp Cys Glu Cys Gly Lys Thr
   100 105 110
   Glu Phe Asn Glu Leu Ile Gln Leu Leu Lys Phe Gln Thr Thr Ile Val
   115 120 125
   Thr Leu Asn Pro Pro Asp Asn Ile Trp Thr Glu Glu Glu Glu Leu Glu
   130 135 140
   Asp Val Pro Trp Phe Pro Arg Lys Ile Ser Glu Leu Asp Arg Cys Ser
   145 150 155 160
   His Arg Val Leu Met Tyr Gly Thr Glu Leu Asp Ala Asp His Pro Gly
   165 170 175
   Phe Lys Asp Asn Val Tyr Arg Gln Arg Arg Lys Tyr Phe Val Asp Val
   180 185 190
   Ala Met Gly Tyr Lys Tyr Gly Gln Pro Ile Pro Arg Val Glu Tyr Thr
   195 200 205
   Glu Glu Glu Thr Lys Thr Trp Gly Val Val Phe Arg Glu Leu Ser Lys
   210 215 220
   Leu Tyr Pro Thr His Ala Cys Arg Glu Tyr Leu Lys Asn Phe Pro Leu
   225 230 235 240
   Leu Thr Lys Tyr Cys Gly Tyr Arg Glu Asp Asn Val Pro Gln Leu Glu
   245 250 255
   Asp Val Ser Met Phe Leu Lys Glu Arg Ser Gly Phe Thr Val Arg Pro
   260 265 270
   Val Ala Gly Tyr Leu Ser Pro Arg Asp Phe Leu Ala Gly Leu Ala Tyr
   275 280 285
   Arg Val Phe His Cys Thr Gln Tyr Val Arg His Gly Ser Asp Pro Leu
       290                 295                 300
   Tyr Thr Pro Glu Pro Asp Thr Cys His Glu Leu Leu Gly His Val Pro
   305                 310                 315                 320
   Leu Leu Ala Asp Pro Lys Phe Ala Gln Phe Ser Gln Glu Ile Gly Leu
                   325                 330                 335
   Ala Ser Leu Gly Ala Ser Asp Glu Asp Val Gln Lys Leu Ala Thr Cys
               340                 345                 350
   Tyr Phe Phe Thr Ile Glu Phe Gly Leu Cys Lys Gln Glu Gly Gln Leu
           355                 360                 365
   Arg Ala Tyr Gly Ala Gly Leu Leu Ser Ser Ile Gly Glu Leu Lys His
       370                 375                 380
   Ala Leu Ser Asp Lys Ala Cys Val Lys Ala Phe Asp Pro Lys Thr Thr
   385                 390                 395                 400
   Cys Leu Gln Glu Cys Leu Ile Thr Thr Phe Gln Asp Ala Tyr Phe Val
                   405                 410                 415
   Ser Glu Ser Phe Glu Glu Ala Lys Glu Lys Met Arg Asp Phe Ala Lys
               420                 425                 430
   Ser Ile Thr Arg Pro Phe Ser Val Tyr Phe Asn Pro Tyr Thr Gln Ser
           435                 440                 445
   Ile Glu Ile Leu Lys Asp Thr Arg Ser Ile Glu Asn Val Val Gln Asp
       450                 455                 460
   Leu Arg Ser Asp Leu Asn Thr Val Cys Asp Ala Leu Asn Lys Met Asn
   465                 470                 475                 480
   Gln Tyr Leu Gly Ile
                   485
<210> 7
   <211> 22
   <212> DNA
   <213> mus musculus
<400> 7
   gacatcggat cagaagactc cc 22
<210> 8
   <211> 21
   <212> DNA
   <213> mus musculus
<400> 8
   ctccctcttt cggaggaatg g 21
<210> 9
   <211> 23
   <212> DNA
   <213> mus musculus
<400> 9
   caccatgatt gaagacaaca agg 23
<210> 10
   <211> 23
   <212> DNA
   <213> mus musculus
<400> 10
   cgtgaattca atcttgggaa tgg 23
<210> 11
   <211> 30
   <212> DNA
   <213> mus musculus
<400> 11
   ggttccctcg gaagatctct gagttagaca 30
<210> 12
   <211> 28
   <212> DNA
   <213> mus musculus
<400> 12
   agagctcccg gaatacaaca ccccaagt 28
<210> 13
   <211> 26
   <212> DNA
   <213> mus musculus
<400> 13
   tgctcttcag caccagggtt ctggac 26
<210> 14
   <211> 25
   <212> DNA
   <213> mus musculus
<400> 14
   agaattgcat gcttactagc caacc 25

## Description

### SUMMARY OF THE INVENTION

The present invention relates to novel, specifically neuronal expressed proteins with tryptophane hydroxylase activity, nucleic acid sequences, recombinant nucleic acid molecules containing these nucleic acid sequences or vectors containing these nucleic acid sequences or the recombinant nucleic acid molecules encoding for a neuronal tryptophane hydroxylase.

The invention also relates to transgenic organisms containing these nucleic acid sequences, the recombinant nucleic acid molecules or the above cited vectors. The invention moreover relates to mono- or polyclonal antibodies directed against the isolated proteins.

Furthermore, the invention relates to the use of these nucleic acid sequences and proteins for diagnosis, predisposition, therapy and monitoring of neuronal diseases. Possible fields of application among others are medicine and the pharmaceutical industry.

### DESCRIPTION

### STATE OF THE ART

Serotonin not only is a neurotransmitter in the central nervous system (CNS), but also a hormone, which is found all over in the periphery and takes part in vasoconstriction and thrombocyte function. Tryptophane hydroxylase is the enzyme determining the velocity of the serotonin biosynthesis (velocity-controlling enzyme). Serotonin (5-hydroxytryptamine, 5-HT) is a monoaminergic neurotransmitter, which is - together with tryptophane hydroxylase (TPH; EC 1.14.16.4) as the velocity-controlling enzyme in the first stage of serotonin synthesis (P.F. Fitzpatrick, Annu. Rev. Biochem. 68, 355 (1999)) - involved in several aspects of mood-control and sleep regulation, of anxiety, alcoholism, drug abuse, food uptake and sexual behaviour (J. Veenstra-VanderWeele, G.M. Anderson, E.H. Cook Jr, Bur. J. Pharmacol. 410, 165 (2000)). The TPH-enzyme, together with phenylalanine (PAH)- and tyrosine hydroxylases (TH), belongs to the superfamily of aromatic amino acid hydroxylases (P.F. Fitzpatrick, Annu. Rev. Biochem. 68, 355 (1999)).

The serotonergic projection system is the most extensive monoaminergic system in the vertebrate brain, however it is also the system the most difficult to investigate. The roots of this system are restricted to a few selectively 5-HT synthesising neurons in the midbrain, the pons and the medulla oblongata, which together represent the different groups of nuclei raphe B1-B9 (A. Dahlström, K. Fuxe, Acta Physiol. Scand. 62: Suppi. 232, 1 (1964)). Moreover, 5-HT constitutes an intermediate in the biosynthesis of the hormone melatonin and therefore the epiphysis has its maximal activity in the dark phase of a 24 hour-regulation, in which it produces the highest amounts of TPH (J.M. Miguez, F.J. Martin, M. Aldegunde, Neurochem. Res. 22, 87 (1997)).

Besides its occurrence in the brain and the epiphysis, TPH was also discovered in enteric neurons (E. Fiorica-Howells, L. Maroteaux, M.D. Gershon, J. Neurosci. 20, 294 (2000)), pre-implanted embryos (D.J. Walther, M. Bader, Mol. Brain Res. 68, 55 (1999)), mast cells (L.M. Finocchiaro et al., J. Interferon Res. 8, 705 (1988)) and - most strikingly - in the enterochromaffin cells of the gastrointestinal tract (L.J. Weber, A. Horita, Biochem. Pharmacol. 14, 1141 (1965)). These cells are expected to be the source of 5-HT in the blood, where it is nearly completely stored in tight storage vesicles of the thrombocytes (J. Champier et al. Lire Sei. 60, 2191 (1997)). 5-HT is involved in different processes in the peripheral tissue, like e.g. regulation of the vascular tonus (M.M. Rapport, A.A. Green, I.H. Page, J. Biol. Chem. 176, 1237 (1948)), intestinal motility (M.D. Gershon, Aliment. Pharmacol. Ther. 13, 15 (1999)), primary haemostasis (J.M. Holland, Proc. Exp. Biol. Med. 151, 32 (1976)) and cell-mediated immune responses (G.P. Geba et al. J. Immunol. 157, 557 (1996)). Since the amount of 5-HT in the brain and the periphery is directly connected with the TPH-activity, the understanding of TPH-expression and -regulation is an important step to clarify the functions of 5-HT in the CNS and in the peripheral tissue.

### DEFICIENCY OF THE STATE OF THE ART

The presence of TPH mRNA and protein in the vertebrate CNS was reported about ten years ago, however the data were inconsistent In the epiphysis and in the nuclei raphe (S. Dumas, M.C. Darmon, J. Delort, J. Mallet, J". Neurosci. Res. 24, 537 (1989); R.P. Hart, R. Yang, L.A. Riley, T.L. Green, Mol. Cell. Neurose!. 2, 71 (1991)) e.g. different mRNA to protein ratios were discovered: comparable TPH-protein levels are present in the nuclei raphe and in the epiphysis, whereas the mRNA-levels are at least 1000 times higher in the latter (F. Chamas, L. Serova, E.L. Sabban, Neurose!. Lett. 267, 157 (1999)). All studies attributed these differences in the protein-mRNA-ratios to different translational efficiencies of mRNAs, which are variably spliced in the 5'-untranslated regions.

However, hints for the existence of different TPH-isoforms (C.D. Cash, Gen. Pharmac. 30, 569 (1998), 21 S.M. Mockkus, K.E. Vrana, J. Mol. Neurose!. 10, 163 (1998)) had been existent for more than 30 years in literature: a) in a purification method, two activity peaks in brain homogenates (H. Nakata, H. Fujisawa, Eur. J. Biochem. 122, 41 (1982)) were discovered and partially purified enzymes with definite biochemical properties were described in dependence of the analysed tissue (D.M. Kühn, M.A. Meyer, W. Lovenberg, Arch. Biochem. Biophys. 199, 355 (1980)), b) the first generation of antibodies against TPH, which was purified from a mouse mastocytoma cell line (P815) did not cross-react with the brain-TPH (H. Nakata, H. Fujisawa, Eur. J. Biochem. 124, 595 (1982)), although the antibody, which currently is commercially available, does cross-react. Until now, these phenomena has been attributed to different phosphorylation states of the proteins (C.D. Cash, Gen. Pharmac. 30, 569 (1998)).

A founded explanation for the mentioned differences of the mRNA- and protein levels or for the contradictory data, however, was missing. This object has been achieved in the present invention.

### CONTEXT OF OBJECTIVE AND SOLUTION

In the present specification, it is shown for the first time by means of "gene targeting", that serotonin is independently synthesised by two different tryptophane hydroxylase isoenzymes in the peripheral tissues and in the neurones. Furthermore, a specifically neuronal isoform of tryptophane hydroxylase is identified for the first time. Although the amino acid sequences of the two tryptophane hydroxylases are highly homologous to each other, the gene sequences only display a little similarity, which explains, why the neuronal tryptophane hydroxylase (in the following designated as snTPH) remained undiscovered for decades. The isolation of the specifically neuronal expressed snTPH can be useful for the development and preparation of novel therapeutics for the treatment of neuronal/psychiatric disorders.

According to this, described herein is a method, by which neuronal and psychiatric diseases can be treated by means of affecting serotonin metabolism via modifying the amount/activity of snTPH. Furthermore, described herein are nucleic acid molecules and isoform specific inhibitors/activators, which can be employed for producing pharmaceutical preparations for the treatment and diagnosis of neuronal disorders, based on affecting the serotonin metabolism by means of snTPH. Further objectives will become obvious from the following description.

These objectives are achieved by the subjects of the independent claims, which are particularly based on providing the DNA sequences for use in medicine according to the invention, the gene products of which can be directly employed for affecting the neuronal serotonin metabolism. Favourable aspects are defined in the dependent claims.

In the present invention, it has - surprisingly and for the first time - been succeeded in providing nucleic acid molecules encoding for a protein with the enzymatic activity of a neuronal tryptophane hydroxylase (snTPH). To investigate the physiolgical effects of a loss of 5-HT-synthesis, mice with a genetic deficiency for TPH were generated. Although a lethal phenotype of this genetic manipulation was expected, as it was determined for animals with a deficiency for TH (Q.Y. Zhou, C.J. Quaife, R.D. Palmiter, Nature 374, 640 (1995)), surprisingly viable homozygous TPH-knock out-mice (TPH(-/-)) were generated. These mice lack 5-HT in the periphery. It was possible to determine for the first time, that the mode of action of 5-HT in primary haemostasis is based on a release of Von-Willebrand-Factor (vWf). Completely unexpected, there was only a slight diminution of stable 5-HT amounts in the classic serotonergic brain regions of TPH(-/-) mice, leading to the identification of a TPH-isoform, which is exclusively expressed in neurones.

### The subject-matter of the herein described invention is defined by the claims.

The present invention thus comprises an isolated nucleic acid sequence for use medicine coding for a polypeptide with neuronal tryptophane hydroxylase activity, selected from a group of:
a) a nucleic acid sequence with the sequence depicted in SEQ ID No: 1, SEQ ID No:3 or SEQ ID No:5,
b) nucleic acid sequences encoding for polypeptides with the biological activity of a neuronal tryptophane hydroxylase which display more than 90% homology to the amino acid sequences given in SEQ ID No:2, SEQ ID No:4 or SEQ ID No:6.

It has to be stated, that the wording "neuronal tryptophane hydroxylase" (sn-TPH) in the text of this specification is meant to include all peptides and proteins with tryptophane hydroxylase activity.

These nucleic acids among eukaryotic and prokaryotic organisms are preferably to be found in mammalians like *Homo sapiens* (human), *Rattus norvegicus* (rat) or *Mus musculus* (mouse).These nucleic acids encode for the amino acid sequences of SEQ ID No:2 (*Homo sapiens*), SEQ ID No:4 (*Mus musculus*) or SEQ ID No:6 (*Rattus norvegicus*). These nucleotide sequences are designated in the following as snTPH- genes and their homologues, the amino acid sequences as snTPH and their homologues.

The nucleic acid sequences described herein encode for the above described proteins, in particular for those with the primary structures shown in SEQ ID No:2, SEQ ID No:4 or SEQ ID No:6. The nucleic acid sequence from *Homo sapiens* is depicted in SEQ ID No:1, that from *Mus musculus* in SEQ ID No:3 and that from *Rattus norvegicus* in SEQ ID No:5. After isolation and sequencing, the nucleic acid sequences according to SEQ ID No: 1, SEQ ID No:3 or SEQ ID No:5 or their functional equivalents like e.g. allelic variants, are available. Allelic variants of SEQ ID No:1, SEQ ID No:3 or SEQ ID No:5 mean variants, which display 6 90 to 100 % homology at the amino acid level (= identity). Allelic variants comprise in particular those functional variants, which are available from the sequences depicted in SEQ ID No:1, SEQ ID No: 3 or SEQ ID No:5 by means of deletion, insertion or substitution of nucleotides, whereby at least one of the essential biological activities is maintained.

Homologous or sequence related nucleic acid sequences can be isolated from all mammalian species including humans by common methods, e.g. homology screening by hybridisation with a probe of the nucleic acids according to the invention or with parts thereof.

Hybridisation in the scope of this invention means a hybridisation under conventional hybridisation conditions, preferably under stringent conditions, as they are e.g. described in Sambrook et al. (Sambrook et al. 1989. Molecular Cloning : A Laboratory Manual, 2. edition, Cold Spring Harbor Laboratory Press, Cold Spring Harbor, New York).

Standard hybridisation conditions e.g. mean - in dependence of the nucleic acid - temperatures between 42°C and 58°C in an aqueous buffer solution with a concentration of 0,1 to 5 X SSC (1 x SSC = 0,15 M NaCl, 15 mM sodium citrate, pH 7,2) or additionally in the presence of 50% formamide like e.g. 42°C in 5 x SSC, 50 % formamide. DNA-sequences hybridising to the DNA-sequences encoding for snTPH can e.g. be isolated from the genomic libraries or cDNA-libraries of any vertebrate, preferably from mammalians, which are in possession of the DNA-sequences according to the invention. Of course, the conditions have sometimes to be suitably adapted in order to distinguish between snTPHs and other TPHs. Such adaption can easily be achieved by standard experiments well known to the skilled artisan. The identification and isolation of such DNA-sequences can e.g. be accomplished by using DNA-sequences comprising exactly or nearly the DNA-sequence depicted in SEQ ID No:1, SEQ ID No:3 or SEQ ID No:5 or parts thereof, or respectively by using the reverse complementary sequences thereof for hybridisation according to standard protocols (e.g. Sambrook et al. (1989), see above). The fragments used as hybridisation probes may also be synthetic fragments produced by means of common techniques of synthesis, whereat the sequence of these fragments largely corresponds to the above mentioned sn-TPH-sequences or parts thereof.

Nucleic acid sequences encoding for a protein with the biological activity of a neuronal tryptophane hydroxylase also comprise DNA-sequences, the nucleic acid sequences of which are degenerated in relation to one of the before mentioned nucleic acid sequences. The degeneration of the genetic code offers the expert - among other aspects - the possibility to adapt the nucleotide sequence of the respective DNA-sequence to the codon preference of the target organisms, thereby optimising gene expression. The DNA-sequences described above also include fragments, derivatives and allelic variants of the above described DNA-sequences encoding for a protein with the biological activity of a neuronal tryptophane hydroxylase. The wording "fragment" in this context designates parts of the DNA-sequence, which are long enough to encode for one of the described proteins.

The wording "derivative" in this context means, that the sequences differs from the above described sequences at one or several positions but displays a high degree of homology. Homology herein denotes a sequence identity of at least over 90 percent. Thereby, the proteins encoded by these DNA-sequences display a sequence identity to the amino acid sequences given in SEQ ID No:2, SEQ ID No:4 or SEQ ID No:6 of at least over 90 percent, 95 percent and 98 percent. The deviations to the above described DNA-sequences may be due to deletion, substitution, insertion or re-combination. The nucleic acid sequences described herein or their fragments may moreover be employed for the isolation of genomic sequences by means of homology screening using the above mentioned hybridisation conditions.

Further described herein are the isolated proteins. These constitute the proteins comprising an amino acid sequence depicted in SEQ ID No: 2, SEQ ID No:4 or SEQ ID No:6 or a sequence, that can be derived therefrom by substitution, inversion, insertion or deletion of one or several amino acid residues, provided that at least one essential biological property of the proteins according to SEQ ID No:2, SEQ ID No:4, SEQ ID No:6 is preserved. Thereby, e.g. specific amino acids may be substituted by amino acids with similar physico-chemical properties (spacial dimensions, basic character, hydrophobic character, etc.). Additionally, one or several amino acids may be exchanged with regard to their position or may be added, deleted or changed by a combination of these measures. The proteins being such modified in respect to SEQ ID No:2, SEQ ID No:4 or SEQ ID No: 6 display at least 90% of sequence identity in relation to the sequences of SEQ ID No:2, SEQ ID No:4 or SEQ ID No:6, whereat the sequence identity is determined according to the algorithm of Altschul et al. (1990, J. Mol. Biol., 215: 403-410, BLAST-programme (current version), determined over the entire length of the proteine sequences).

The wording "functional equivalents" of the mentioned sequences designates nucleic acids encoding for proteins possessing the biological activity of snTPH as described herein, and at least a 50% activity compared to the sequences shown in SEQ ID No:2, SEQ ID No:4 or SEQ ID No:6. These functional equivalents preferably interact with other proteins by forming so-called protein complexes (protein heteromers) therewith.

Also described is the specific binding of synthetic or natural agonists and antagonists to the proteins according to the disclosure (the proteins possessing the amino acid sequences of SEQ ID No:2, SEQ ID No:4 or SEQ ID No:6 or their functional equivalents) constitutes a part of the proteins' essential biological properties, which can be altered or modified in consequence of this binding behaviour.

The proteins according to the invention are advantageously isolated from the brain of mammalian species like *Homo sapiens* (human SCLC-cells, SHP-77 and other examples), *Mus musculus* or *Rattus norvegicus.* Moreover, the disclosure refers to nucleic acid molecules, which comprise the nucleic acid sequences described or which have been produced or deduced from these sequences by means of naturally occurring or genetic or chemical processes or synthesis methods. Possible examples for these molecules are DNA- or RNA-molecules, cDNA, genomic DNA, mRNA, etc.

It is of further advantage to functionally combine the nucleic acids described herein with at least one genetic regulatory element in order to generate the recombinant nucleic acid molecules (expression cassette, gene construct), which enable transcription and - if desired - translation within a cell. To this aim, the nucleic acid sequences are in general functionally connected to genetic regulatory elements like transcriptional or translational signals. Thereby, the nucleic acid sequence may be connected in the antisense- or sense-direction to one or several regulatory signals. According to the intended application, this combination may lead to an increased or decreased gene expression.

In principle, all naturally occurring promoters and their regulatory sequences are suitable for expressing the DNA-sequences comprised within the recombinant nucleic acid molecules described herein in eukaryotic or prokaryotic cells.

The regulatory sequences or factors may thereby preferably affect the expression in a positive manner leading to an increase of expression. An enforcement of the regulatory elements can e.g. be realised advantageously at the level of transcription by providing strong transcriptional signals like promotors. Another favourable possibility is a targeted genetic modification of the promotor occurring naturally upstream of the respective sequences. Additional regulatory signals like enhancers or polyadenylation signals or terminators positioned at the 3' site may be functionally employed in the recombinant nucleic acid molecules.

In any case, the expert can find suitable promoters in literature or can isolate them from arbitrary organisms by means of standard methods.

Furthermore, transcriptional or terminating sequences are present, which may serve to properly terminate transcription or to add a poly(A)-tail to the transcript, which is believed to have a function in stabilising the transcript. Elements of this kind have been described in literature and can be arbitrarily exchanged.

Moreover, the invention refers to the provision of vectors for use in medicine comprising the nucleic acid sequences described herein or a recombinant nucleic acid molecule described herein , the employment of which serves to create transgenic organisms with increased or decreased production/activity of snTPH.

Further disclosed are to vectors, in particular te plasmids, cosmids, viruses, bacteriophages and other vectors commonly used in genetic engineer-ing, whereby these vectors comprise the before mentioned nucleic acid molecules described herein and can - if desired - be used for transferring the nucleic acid molecules described herein to eukaryotic or prokaryotic cells.

Moreover, the recombinant nucleic acid molecule or the nucleic acids according to the disclosure may also be expressed in the form of fragments, which are suited for therapeutic or diagnostic applications. In order to generate the recombinant proteins, vector systems or oligonucleotides can be employed, which elongate the nucleic acid or the recombinant nucleic acid molecule by adding specific nucleotide sequences, thereby encoding for modified polypeptides, the purification of which is thereby facilitated (addition of TAGs).

As a host organism, in principle all organisms are suitable, which allow an expression of the nucleic acids described herein the allelic variants thereof, the homologues thereof, the functional equivalents or derivatives thereof, the recombinant nucleic acid molecule or the vectors. The wording "host organism" is to be understood as including e.g. eukaryotic or prokaryotic host organisms like bacteria, fungi, yeasts, plant cells or animal cells. Preferred organisms are bacteria like *Escherichia coli, Streptomyces, Bacillus* or *Pseudomonas,* eukaryotic microorganisms like *Saccharomyces cerevisiae, Aspergillus,* higher eukaryotic cells from human or animals like e.g. the cell lines COS7, NG108-15 and P815.

These transgenic or recombinant organisms are characterised by the feature, that they either - as natural organisms - do not contain the nucleic acids described herein, the allelic variants thereof, the homologues thereof, the functional equivalents or derivatives, the recombinant nucleic acid molecule or the vectors; or that they do not contain these elements at this site in the genome or in the cell; or that the natural promoter of the recombinant nucleic acid molecules described herein was genetically modified such, that the respective genes are accordingly expressed in an increased or decreased manner.

Thereby, the described host organism can contain at least one nucleic acid sequence described herein or at least one recombinant nucleic acid molecule or at least one vector described herein.

Preferably, the gene product can also be expressed in transgenic organisms like transgenic animals, e.g. in mice, rats, cheeps, bovine animals or pigs. Also transgenic plants are contemplated, whereat proteins may be yielded as a result of molecular farming. In the case of transgenic organisms, also the so-called "knock-out-animals" are contemplated.

Thereby, the transgenic animals may contain a functional or non-functional nucleic acid sequence described herein or a functional or non-functional nucleic acid construct.

Disclosed is the use of the described nucleic acids or of parts thereof for the purpose of genetic therapy in mammals, e.g. in humans. Also sequences, which are complementary to the nucleic acids according to the invention or to parts thereof, can be employed for genetic therapy. Genetic therapy thereby comprises all therapeutic methods, which either introduce sequences described herein into the body or parts of the body or which affect the expression of sequences described herein. To this aim, one can employ oligonucleotides like e.g. antisense-oligonucleotides or RNA-DNA hybrid oligonucleotides with arbitrary modifications, which comprise parts of the sequences according to the present disclosure. Also viral constructs containing a sequence as described or parts thereof can be employed.

A further advantageous embodiment is the use of the nucleic acid sequences described herein, the recombinant nucleic acid molecules described herein or the amino acid sequence described herein for the identification, discovery or detection of proteins, which exhibit specific binding affinities for a protein characterised by the polypeptide (i.e. amino acid sequence) described herein or for the identification of nucleic acids encoding for proteins with specific binding affinities for amino acid sequences described herein. The Two-Hybrid System or other biochemical methods may be used for this purpose either alone or in combination. Thereby, interaction domains of the proteins according to the disclosure and other conserved regions and thus potential pharmacological targets can be identified.

Further disclosed is the use of the Two-Hybrid System or of other biochemical methods alone or in combination for identifying the interaction domains of snTPH and the application for pharmacotherapeutic use.

Further disclosed is the use of a nucleic acid sequence described herein as a marker for human hereditary diseases, in particular neuronal diseases.

A further aspect of the present invention is the use of a protein described herein as an antigen for producing specific polyclonal or monoclonal antibodies or antibody mixtures directed against proteins described herein. The wording "antibody" thereby comprises polyclonal, monoclonal, human or humanised or recombinant antibodies or fragments thereof, single chain antibodies and also synthetic antibodies. The antibodies for use in medicine according to the invention or their fragments in principle refer to all immune globulin classes or their subclasses or their mixtures. As examples for fragments, all truncated or modified antibody fragments with one or two binding sites complementary to the antigen are to be mentioned. Also genetically modified non-truncated fragments may be advantageously employed. The antibodies or fragments can be used alone or in mixtures. The antibody genes for genetic procedures can e.g. be isolated from the hybridoma cells in a way familiar to the expert. To this aim, antibody-producing cells are grown and, after the cells have reached a sufficient optical density, the mRNA is isolated from the cells in a well known manner by cell lysis with guanidine thiocyanate, acidification with sodium acetate, extraction with phenole, chloroform/iso-amyl alcohol, precipitation with isopropanol and washing with ethanol, followed by the synthesis of cDNA from the mRNA by means of reverse transcriptase. The synthesised cDNA may be directly used for vector insertion or expression or modified by genetic manipulation, e.g. by site directed mutagenesis, introduction of insertions, inversions, deletions or nucleotide base exchanges, followed by an introduction into suitable animal, fungal, bacterial or viral vectors and an expression in the respective host organisms.

Specific antibodies against the proteins described herein can be suitable both as diagnostic agents and as therapeutics in disease conditions, which are - among other features - characterised by changes in the serotonin metabolism.

The nucleic acid sequences described herein, their functional equivalents, homologues, derivatives or fragments, the proteins encoded by them and comprising the amino acid sequences described herein as well as the reagents derived therefrom (oligonucleotides, antibodies, peptides) can be used for detecting a predisposition, for diagnosis, for therapy and for monitoring of neurological diseases, in particular in the context of serotonergic effects in behavioural physiology.

Affecting the snTPH can be accomplished in the following ways:
Specific inhibitors of neuronal TPH-isoforms are developed, which are based on the specific molecular differences of the TPH-isoforms. Also developed are inhibitors, which are not permeable for the blood-brain barrier. Specific inhibitors for the TPH-isoforms can be identified in vitro by standard screening methods familiar to the expert, since the identified cDNAs of snTPH can be used in expression systems in order to specifically express the pure isoforms.

These inhibitors - besides their diagnostic benefits - also offer therapeutic applications, since increased serotonin levels in the CNS are found in a plurality of medical complications.

Another aspect of the present invention therefore relates to a method for isolating a compound that binds to a polypeptide described herein, comprising the steps of: (a) contacting a mammalian cell which expresses a polypeptide described herein having sn-TPH activity with a compound; (b) detecting the presence of the compound which binds to the sn-TPH polypeptide. Such so-called "binding-assays" are standard practice in the art and can include suitably labelled, e.g. radioactively labelled, dye-labelled, enzyme labelled, antigen labelled and/or mass-tag labelled putative binding compounds that can be detected using the respective suitable agent, e.g. antibodies, enzyme substrates, and the like.

Further disclosed is a method for the production of a pharmaceutical composition comprising the steps of the method as given above and the subsequent step of formulating the compound identified in step (c) and/or its pharmaceutically acceptable salts in a pharmaceutically acceptable form. Suitable formulations depend *inter alia* from the route of administration and can easily be produced by the person of skill, based on the information as given in the respective literature. These pharmaceutical compositions may be administered orally, rectally, parenterally, intracistemally, intravaginally, intraperitoneally, topically (as by powders, ointments, drops or transdermal patch), bucally, or as an oral or nasal spray.

As used herein, "pharmaceutically acceptable carrier" is intended to mean a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion. One of ordinary skill will recognise that the choice of a particular mode of administration can be made empirically based upon considerations such as the particular disease state being treated; the type and degree of the response to be achieved; the specific agent or composition employed; the age, body weight, general health, sex and diet of the patient; the time of administration and rate of excretion of the agent or composition; the duration of the treatment; drugs (such as a chemotherapeutic agent) used in combination or coincidental with the specific composition; and like factors well known in the medical arts.

Pharmaceutical compositions for parenteral injection may comprise pharmaceutically acceptable sterile aqueous or nonaqueous solutions, dispersions, suspensions or emulsions as well as sterile powders for reconstitution into sterile injectable solutions or dispersions just prior to use. Illustrative examples of suitable aqueous and nonaqueous carriers, diluents, solvents or vehicles include, but are not limited to, water, ethanol, polyols (such as glycerol, propylene glycol, polyethylene glycol, and the like), carboxymethylcelulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The compositions may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminium monostearate and gelatine.

Affecting the snTPH-regulation (activity and/or amount of snTPH) may be accomplished in the following ways :
A specific down-regulation of snTPH can be accomplished biochemically by means of ri-bozymes, antisense-oligonucleotides or by means of antisense-RNA expression, whereat the sequence differences of the isoform mRNAs allow to influence just one mRNA. Furthermore, also pharmacological approaches based on specific TPH-inhibitors like e.g. p-chlorophenylalanine or p-ethinylphenylalanine can be employed according to the invention.

The serotonin production is stimulated in a molecular biological manner preferably by tissue-specific overexpression of snTPH. In the pharmacological approach, both the precursor substance, 5-hydroxy-tryptophane and substituted analogues can be applied, as well as serotonin itself.

The method for diagnosing neuronal diseases is characterised thereby, that a specific inhibition of the peripheral serotonin biosynthesis is accomplished, which is followed by measuring the metabolite concentration stemming from the CNS and by determining the severity of the disease by means of a comparative graph. To this aim, it is necessary to employ substances, which cannot cross the blood-brain barrier.

Another aspect of the present disclosure is related to a method for determining the pharmacogenetic properties of a pharmaceutically active compound, comprising a) administering the compound to a mammal, b) determining the level of expression of snTPH in a biological sample obtained from said mammal, and c) comparing said level of expression of snTPH with a level obtained from a control sample. A "pharmaceutically active compound" is a compound that alleviates the symptoms of a medical condition related to the level of expression or the function of snTPH, either alone or in combination with other compounds, such as, for example, used in the combination therapeutic as described herein. The pharmaceutically active compound can be derived from so-called "small molecules" (i.e. having a molecular weight of less than 500 kDa) or larger molecules, such as peptides, proteins, antibodies, oligonucleotides, and the like.

Further described is a method for the improved treatment of a disease, comprising performing the method as described above, and increasing or decreasing the doses of the pharmaceutically active compound to be applied to said patient. The inclusion of specifically derived pharmacogenetic information in the treatment of a patient is a particular aspect of the so-called "personalised-medicine". Personalised treatment proves to be more efficient than the general "standard" application of a doses of a medicament (i.e. containing a pharmaceutically active compound) (see, for example, Kalow W. Pharmacogenetics and personalised medicine. Fundam Clin Pharmacol. 2002 Oct;16(5):337-42; Jain KK. Personalised medicine. Curr Opin Mol Ther. 2002 Dec;4(6):548-58; Ross JS, Ginsburg GS. Integrating diagnostics and therapeutics: revolutionising drug discovery and patient care. Drug Discov Today. 2002 Aug 15;7(16):859-64; Ross JS, Ginsburg GS. The integration of molecular diagnostics with therapeutics. Implications for drug development and pathology practice. Am J Clin Pathol. 2003 Jan;119(1):26-36; Ikeda M, Kitajima T, Iwata N, Ozaki N. Molecular genetics of mood disorders, Nihon Shinkei Seishin Yakuri-gaku Zasshi. 2002 Oct;22(5):137-43; Pagliarulo V, Datar RH, Cote RJ. Role of genetic and expression profiling in pharmacogenomics: the changing face of patient management. Curr Issues Mol Biol 2002 Oct;4(4):101-10; Iida A, Saito S, Sekine A, Nakamura Y. SNP collection, pharmacogenomics, and the future of drug therapy Gan To Kagaku Ryoho. 2002 Sep;29(9):1665-73; Smith DJ, Lusis AJ. The allelic structure of common disease. Hum Mol Genet. 2002 Oct 1;11(20):2455-61.).

The above-indicated methods can be used in the diagnosis and/or treatment of diseases that are selected from neuronal diseases, such as sleep disturbances, anxiety, alcoholism, drug abuse, disorders of food uptake and/or sexual disorders.

Furthermore, it is advantageous to use the described nucleic acid sequences , the described recombinant nucleic acid molecules or the described amino acid sequences for the treatment of sleep disturbances, anxiety, alcoholism, drug abuse, abnormal food intake or sexual disturbances, characterised in that the serotonin level is modified by modulating the gene expression of snTPH or by modifying the snTPH activity.

The nucleic acid sequences according to the disclosure, the recombinant nucleic acid molecules according to the disclosure or the amino acid sequences according to the disclosure are used for the development and production of a combination therapeutic, which comprises a protein according to the present disclosure and at least one further protein, in particular for the regulation of the serotonin metabolism. Besides a common carrier material, the combination therapeutic can in principle comprise each pharmaceutically well-tolerated protein. The combination therapeutic as a further active substance contains a peripheral tryptophane hydroxylase for the simultaneous treatment of neuronal/psychiatric diseases and thrombotic diseases like coronary infarction and stroke (the peripheral and the neuronal production of serotonin are simultaneously affected in the form of an increase or decrease). In this context, it should be mentioned, that the use of SSRI (serotonin specific reuptake inhibitor)-antidepressants in psycho-pharmacological therapy may in some cases lead to severe bleeding complications. This can be prevented by the employment of a preparation with specific neuronal effect. Accordingly, the described combination therapeutic is for the treatment of bleeding episodes in the psycho-pharmacological treatment of depressions with antidepressants affecting the serotonin reuptake transporter, comprising antidepressants and von Willebrand-factor.

### EXAMPLES

The described invention will be further explained hereafter by means of the following examples. The expert will recognise various other embodiments from the present description. It is thus explicitly stated, that the examples, the description of the figures and the figures merely have the purpose of elucidation and are not to be understood as restrictions of the invention.

In case of not being otherwise indicated, the experimental procedure was accomplished according to the protocols of Ausubel et al. (eds.), 1998, Current Protocols in Molecular Biology. John Wiley & Sons, New York and Sambrook et al. (eds.), 1989, Molecular cloning: A laboratory manual. CSH Laboratory Press, New York.

### Molecular Biological Methods and Generation of Tph-/- Mice.

All cloning procedures, PCR, and Southern blotting were conducted according to standard protocols (J. Sambrook, E. F. Fritsch, T. Maniatis, Molecular Cloning: A Laboratory Manual. 2nd edn.: 1pp (1989)). For the generation of a targeting vector, a 2.2 kb and a 8.6 kb amplicon were obtained with long range PCR using primers derived from the published *Tph* sequence of the mouse (J. Stoll, C. A. Kozak, D. Goldman, Genomics 7, 88 (1990)) (5'-GACATCGGATCAGAAGACTCCC-3' (SEQ ID No. 7)/5'-CTCCCTCTTTCGGAGGAATGG-3' (SEQ ID No. 8), and 5'-CACCATGATTGAAGACAACAAGG-3' (SEQ ID No. 9)/5'-CGTGAATTCAATCTTGGGAATGG-3' (SEQ ID No. 10), respectively). The 2.2 kb fragment was cloned into pCRII-T-vector (Invitrogen, Leek, NL) to obtain pIn1c2, and subsequently sequenced. As short arm of the targeting vector, a 1 kb Fragment was isolated by *Hind* III digestion and T4-DNA polymerase blunting, followed by *Xho* I digestion. To obtain a long arm for the targeting vector, the 8.6 kb fragment was first partially digested with *BamH* I, and then digested with *Spe* I, and the resulting 5.2 kb fragment was isolated. The 1 kb and the 5.2 kb fragments were then cloned into a vector containing a neomycin-resistance cassette and a TK-cassette. ES-cell targeting with this vector and embryo manipulations were carried out according to standard protocols (T. Walther et al. J. Biol. Chem. 273, 11867 (1998)). Southern blot of *Eco* RI-digested genomic DNA using either a internal or a external probe detects a 10 kb *Eco* RI fragment in wild-type animals, whereas in KO-animals a shorter 8 kb fragment is detected due to the Eco RI site contained in the neomycin resistance cassette.

Total RNA preparation and ribonuclease protection assays (RPA) were performed using TRIZOL reagent (Gibco, Eggenheim, Germany) and the RPAII kit (Ambion, Austin, TX) as previously described (D. J. Walther, M. Bader, Mol. Brain Res. 68, 55 (1999)). Radioactivity was visualized by a BAS2000 phosphoimager system (Fuji, Tokyo, Japan). For *Tph1* we used a previously described RPA probe (D. J. Walther, M. Bader, Mol. Brain Res. 68, 55 (1999)). Furthermore, we generated a RPA probe for *Tph2* with 5'-GGTTCCCTCGGAAGATCTCTGAGTTAGACA-3' sense (SEQ ID No. 11) and 5'-AGAGCTCCCGGAATACAACACCCCAAGT-3' antisense (SEQ ID No. 12) primers corresponding to parts of *Tph2* exons 4 and 6, respectively.

For expression studies, we amplified the complete coding sequence with 5'-TGCTCTTCAGCACCAGGGTTCTGGAC-3' sense (SEQ ID No. 13) and 5'-AGAATTGCATGCTTACTAGCCAACC-3' antisense (SEQ ID No. 14) primers and cloned the cDNA into a eukaryotic expression vector under control of a CMV promotor. We transiently transfected COS7 cells with this vector using the Ca₃(PO₄)₂ coprecipitation method (J. Sambrook, E. F. Fritsch, T. Maniatis, Molecular Cloning: A Laboratory Manual. 2nd edn.: 1pp (1989)). Cell culture was conducted as described (D. J. Walther, J.-U. Peter, M. Bader, Cancer 94, 3135 (2002)).

### TPH Activity Assay.

The activity of cell homogenates was determined using an adapted method basically as described (E. M. Gal, K. Patterson, Analyt. Biochem. 52, 625 (1973)), monitoring for 5-HTP accumulation by HPLC in presence of NSD1015, a inhibitor of aromatic amino acid decar-boxylases, in the reaction mixture. Briefly: cells were harvested with a scraper and washed twice with phosphate buffered saline, resuspended in 75 mM tris-acetate buffer (pH 7.5), and lysed by sonication. After withdrawal of an aliquot for protein determination, the homogenates were immediately pre-incubated in 100 µl buffer containing 2 mg/ml catalase, 25 mM DTT and 100 µM Fe(NH4)2(SO4)2 for 10 min at 30 °C in the dark. The pre-incubated samples were incubated at 37 °C for 30 min after addition of 400 µl 15 mM tris-acetate buffer (pH 6.4) containing a final concentration of 300 µM L-Trp and 300 µM 6-methyl-tetrahydropterin and 2 mM NSD 1015. The reaction was terminated by addition of perchloric acid to a final concentration of 300 mM. All reagents were purchased from Sigma, Deisen-hofen, Germany.

### Analytical HPLC Methods.

The brains were rapidly removed, immediately frozen on dry ice and stored at -80°C until use. The hippocampus and frontal cortex were dissected from the frozen brain on a cold plate (-10°C) according to Paxinos and Watson (G. Paxinos, C. Watson, The Rat Brain In Stereotactic Coordinates. Academic Press, Inc., NY, 1 (1997)). Tissue extracts for the determination of 5-HT, Trp, and 5-HIAA contents were obtained and analysed using reverse phase HPLC with fluorometric detection (BPLC-FD) as previously described (D. J. Walther, M. Bader, Mol. Brain Res 68, 55 (1999)), or analysed using a HPLC coupled with electrochemical detection (HPLCECD; Decade, Antec Leyden BV, Leiden, The Netherlands) as previously described (G. Sperk, J. Neurochem. 38, 840 (1982)).

### Behavioural Studies.

All behavioural studies were conducted as previously described (T. Walther et al. J. Biol. Chem. 273, 11867 (1998), H. Thomas, H. Fink, T. R. Sohr, M. Voits, Pharmacol. Biochem. Behav. 65, 15 (2000), P. Gerhardt, M. Voits, H. Fink, J. P. Huston, Peptides 15, 689 (1994), R. U. Hasenöhrl, C. Frisch, S. Nikolaus, J. P. Huston, Behav. Neural Biol. 62, 110 (1994)) using 11 to 12 animals of each genotype.

**Table 1: Behavioural parameters of Tph-/- mice.**

| **Elevated Plus Maze (10 min)** | | | |
|---|---|---|---|
| | *Tph*+/+ (n=11) | *Tph-*/*-* (n=12) | |
| total distance travelled (m) | 5.5 ± 0.6 | 5.8 ± 0.4 | (n.s.) |
| total no. of entries | 27.8 ± 2.9 | 21.9 ± 3.5 | (n.s.) |
| % entries open of total no. of entries | 18.3 ± 2.6 | 19.0 ± 2.4 | (n.s.) |
| % time open of total time in zones | 5.6 ± 2.0 | 4.2 ± 0.8 | (n.s.) |

| **Hole Board (10 min)** | | | |
|---|---|---|---|
| | *Tph*+/+ (n=12) | *Tph*-/- (n=12) | |
| movement time (sec) | | | |
| day 1 | 388.8 ± 20.2 | 392.7 ± 19.8 | (n.s.) |
| day 2 | 289.3 ± 32.2* | 304.8 ± 19.6* | (n.s.) |
| distance (cm) | | | |
| day 1 | 797.2 ± 75.7 | 883.2 ± 78.8 | (ns.) |
| day 2 | 522.6 ± 93.9* | 581.5 ± 63.0* | (n.s.) |
| no. of nosepokes | | | |
| day 1 | 8.0 ± 1.3 | 6.3 ± 0.8 | (n.s.) |
| day 2 | 3.6 ± 0.9* | 1.9 ± 0.5* | (n.s.) |
| nosepokes - cumulative time | | | |
| day 1 | 9.8 ± 1.7 | 8.1 ± 1.1 | (n.s.) |
| day 2 | 4.0 ±1.0* | 2.8 ± 0.7* | (n.s.) |

| | | | |
|---|---|---|---|
| Numbers are the mean values ± SEM; n.s.: not significant; *: p<0.05 vs. day 1. | | | |

**GenBank Accession Numbers.** The following *Tph* sequences can be found in databases: Chicken *Tph1* P70080, chicken *Tph2* (partial) EST AL584678. Zebrafish *Tph1* (partial) EST BI475520, zebrafish *Tph2* (partial) EST BI979362. Mouse *Tph1* P17532, mouse *Tph2* new entry AY090565. Rat *Tph1* P09810, rat *Tph2* new entry AY098915. Human *Tph1* P17752, human *Tph2* new entry AY098914.

### UNCHANGED 5-HT BIOSYNTHESIS IN THE CNS OF TPH(-/-)-MICE

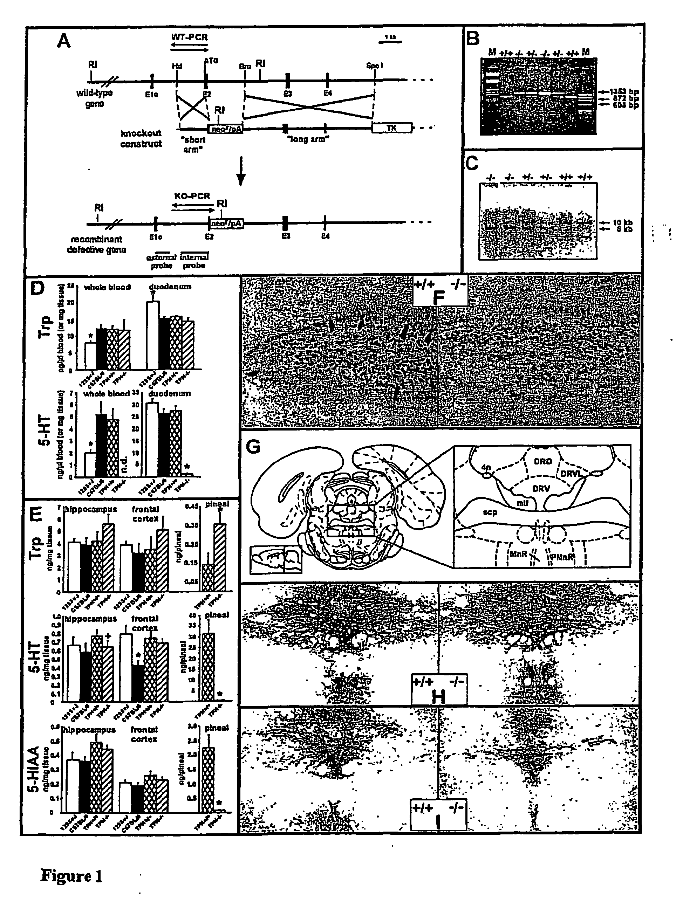

A "gene targeting"-vector was created, which substituted the 3'-coding region of the first translated exon of the *tph*-gene by a transcriptionally active gene cassette conferring a neomycin resistance in order to inactivate TPH-expression (Fig. 1A). Mice with a TPH-deficiency were generated by means of standard protocols for gene targeting (Walther et al., J. Biol. Chem. 273, 11867 (1998)). Animals being heterozygous or homozygous for the mutated *tph-*allele were identified by PCR (Fig. 1B) and Southern Blots with an outer (Fig. 1C) and an inner probe, which detected identical fragments.

The loss of TPH-activity in "gene targeted"-mice was first demonstrated in whole blood samples by using high pressure liquid chromatography with a fluorimetric (HPLC-FD; Fig. 1D) or electrochemical (HPLC-ECD; Fig. 1D and E) detection. The wild type TPH(+/+)-offspring of one pair or mice parents and the inbred lines C57BL/6 and 129SvJ, of which the TPH(-/-)-animals descended, contained the expected high concentrations of 5-HT, whereas in TPH(-/-)-animals no 5-HT was detectable with these sensitive methods. Since the minimum detection limit in whole blood was 5pg/U, the 5-HT must have been less than 1% of the normal concentration in mouse thrombocytes.

Moreover, gastrointestinal enterochromaffin cells, which constitute the source of the large 5-HT pools in whole blood, were free of 5-HT in TPH(-/-)-mice, as it was shown by immuno-histochemistry (Fig. IF), whereas distinct traces of 5-HT (less than 4% of the normal level) were surprisingly found in the duodenum of TPH(-/-)-mice when using HPLC for detection (Fig. 1D).

It is extremely remarkable, that only a little decrease of 5-HT-amounts and its most important metabolite 5-hydroxyindole-3-acetic acid (5-HIAA) were detected in the serotonergic projection areas of TPH(-/-)-mice, whereat this decrease in the hippocampus only reached statistical significance in comparison to TPH(+/+)-mice (Fig. 1E). The differences of 5-HT amounts between two laboratory mice races (C57/BL/6 and 129SvJ) were larger than the differences of 5-HT amounts found in the analysed brain regions of TPH(-/-)- and TPH(+/+)-mice. As a striking result however, the epiphysis of TPH(-/-)-mice contained less than 1% of the 5-HT and 5-HIAA found in TPH(+/+)-mice (Fig. 1E).

The presence of 5-HT and TPH in the CNS was further analysed on a qualitative level by immunohistochemical methods. In the nuclei raphe (Fig. 1G) only minimal differences in 5-HT amounts (Fig. 1H) and TPH-expression (Fig. 1I) were detectable. In consequence, the targeting of the *tph*-gene mirrored an unexpected separation between the 5-HT-biosynthesis in the CNS and in the peripheral tissue.

In order to analyse the effects of the marginally altered 5-HT-amounts, behavioural experiments were performed with TPH(-/-)-mice and control mice. When using the "Elevated-plus-maze"-, the "Hole board"- and the "Water-maze"- tests, which indicate a behaviour associated with 5-HT, no significant differences between the behaviour of the animals with the different genotypes were detected.

### IDENTIFICATION OF A NEURONAL TPH

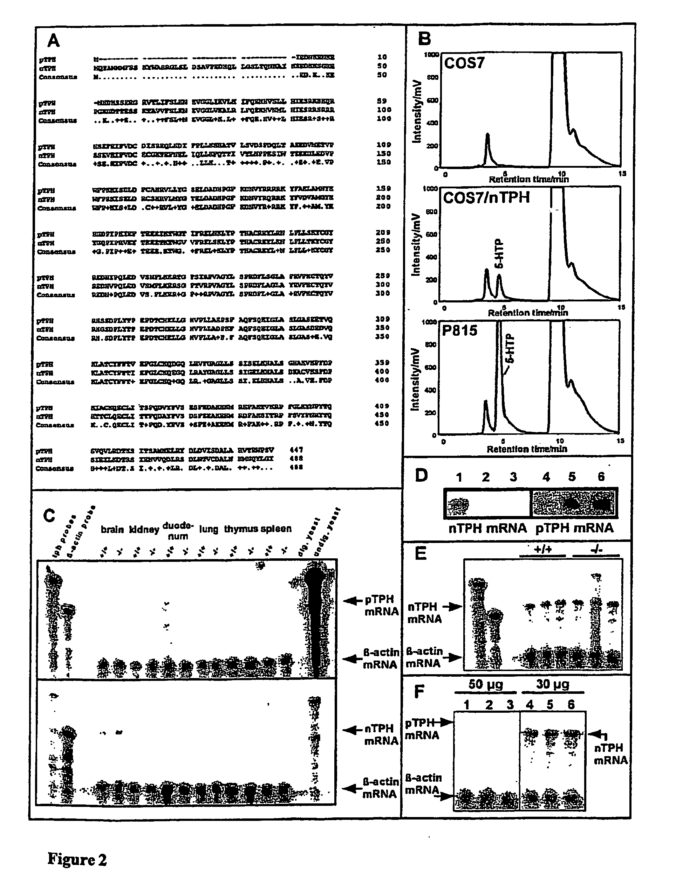

Starting from the findings described in example 1, the reason for the maintained TPH-expression in the brain of the TPH(-/-)-animals was investigated. The existence of a second, not yet discovered genetic locus encoding for a neurospecific TPH-isoform was presumed, since the immunohistochemical experiments (Fig. 2) and the Western Blots (data not shown) indicated the presence of a TPH-protein in the brain of the TPH(-/-)-animals, which had a weight similar to the known TPH.

Upon screening the High Throughput Genomic Sequences (HTGS) of the database GenBank by using short translated TPH-sequences, a human genomic clone derived from chromosome 12 (GenBank accession: AC023966) was identified. This clone contained an open reading frame, which is very similar to the TPH exon 4, but different to PAH, which is also located on chromosome 12. A further analysis of the sequence revealed the presence of other putative exons. Moreover, a genomic rat clone (AC099197) was identified, which also contained the putative exons. Primers were developed, which are based on parts of these sequences, which are homologous to the exons 4 and 6 of mouse-TPH. These primers led to a cDNA-fragment derived from the brain of TPH(-/-)-mice, whereat this cDNA-fragment differed from known mouse forms of TPH, PAH and TH. Starting from the sequence of this fragment, the full-length cDNA of neuronal TPH was determined by using 5'- and 3'-RACE (novel GenBank entry for the neuronal TPH: AY090565). The derived amino acid sequence of this enzyme shows an homology of 86% in relation to the classical mouse-TPH sequence (Fig. 2A).

This cDNA was cloned into a eukaryotic expression vector and COSV-cells lacking an endogenous TPH activity were transiently transfected. Homogenates of transfected cells show a tryptophane hydroxylase activity confirming the specificity of this brain enzyme (Fig. 2B).

By means of RT-PCR, it was possible to confirm the expression of this second TPH-isoform in rats (novel GenBank entry: AY098915) and to identify a homologous EST-sequence in chickens (AL584678), which is different from known chicken-TPH (GGU26428). The RT-PCR analysis also revealed EST-sequences from the zebra fish brain (BI475520, BI981676 and BM025115) besides a further EST-sequence from the zebra fish ovary (BI979302), the latter displaying a high homology to known TPH. Thus, a cell-specific and mutually exclusive expression of these two TPH-isoforms seems to occur in vertebrates. Moreover, also the human homologue of the new TPH-isoform (novel GenBank entry: AY098914) was cloned and the sequence determined.

The notorious TPH mRNA was detected in the duodenum and also in the thymus gland and in the spleen, but not in the brain, when 20 µg total RNA were used in RNase protection assays being specific for the two TPH-isoforms (Fig. 2C). In contrast, the neuronal TPH is only detectable in the brain, where it is strongly expressed (Fig. 2C). Therefore, the classical TPH is designated as peripheral TPH (pTPH) and the novel enzyme as neuronal TPH (snTPH).

Furthermore, also the analysis of P815 mRNA supports a mutually exclusive cell-specific expression of snTPH and pTPH, since only pTPH mRNA, but not snTPH mRNA was successfully determined by specific RNase protection essays (RPA, Fig. 2D), and it was additionally found, why anti-TPH anti-sera produced against P815 TPH were not able to detect the enzyme derived from the brain stem. According to the mutually exclusive, cell-specific expression, it is shown in this specification, that the amounts of snTPH mRNA are not affected in the brain of the TPH(-/-)-mice (Fig. 2E). Moreover, the mid-brain and the pons - in all RNA-samples of control animals used in the RPAs - exhibited an amount of snTPH mRNA about 50 times greater than that of pTPH mRNA (Fig. 2F).

The results obtained from TPH(-/-)-mice thus show, that 5-HT is synthesised in the CNS and in the peripheral tissue by two independently controlled serotonergic systems, defined by two TPH-isoforms, which are encoded by different genes with tissue-specific and mutually exclusive expression patterns, thus readily explaining a great amount of the mysterious former data.

### Description of the sequences and figures

SEQ ID No:1: The c-DNA nucleic acid sequence of neuronal tryptophane hydroxylase from *Homo sapiens,*
SEQ ID No:2: The amino acid sequence of neuronal tryptophane hydroxylase from *Homo sapiens,*
SEQ ID No:3: The c-DNA nucleic acid sequence of neuronal tryptophane hydroxylase from Mus musculus,
SEQ ID No:4: The amino acid sequence of neuronal tryptophane hydroxylase from *Mus mus*culus,
SEQ ID No:5: The c-DNA nucleic acid sequence of neuronal tryptophane hydroxylase from *Rattus norvegicus,*
SEQ ID No:6: The amino acid sequence of neuronal tryptophane hydroxylase from *Rattus norvegicus,*
SEQ ID No:7 to SEQ ID No:10: Long range PCR primers as employed in the examples,
SEQ ID No:11 to SEQ ID No: 12: RPA probe primers for *Tph2* as employed in the examples, and
SEQ ID No:13 to SEQ ID No: 14: sense and antisense primers as employed in the examples.

Figure 1:
   Generation of TPH(-/-)-mice; quantitative analysis of Trp and 5-HT in the peripheral tissue and quantitative analysis of Trp, 5-HT and 5-HIAA in selected brain regions using HPLC-FD and immunohistochemical staining.
   (A) Schematic presentation of the targeting procedure with an indication of the first 4 of 11 exons of the TPH-gene. The knock out-construct (KO) was integrated in one allele of ES-cells by means of homologous recombination, leading to a destruction of the first coding exon (exon 2) of the TPH-gene. Furthermore, the integrated neomycin-resistance cassette (neo^{r}) also comprises a transcriptional stop signal (pA). The positions of the analytical PCR-amplicons for identifying the wildtype and the KO-allele are indicated, as well as the positions of inner and outer probes and the EcoRI-sites (boldface: RI), which were used for the Southern Blot. Restriction sites: RI: EcoRI; Hd: HindIII; Bm: BamHI; Sc: SacI.
   (B) Agarose gel electrophoresis of analytical PCR-products. As indicated in (A), the PCR experiments detect a 1,1 kb-Fragment in wildtype animals, whereas the KO-allele is detected as a 1,3 kb-fragment.
   (C) Southern Blot of EcoRI-digested genomic DNA. As indicated in (A), both probes detect an EcoRI-fragment of 10 kb in the wildtype animals, whereas - due to the EcoRI-site in the neomycin resistance cassette - a shorter 8 kb-fragment is detected in the KO-animals.
   (D) Trp and 5-HT in whole blood and the duodenum of the following mice lines and mutants: 129vJ, C57BL/6, TPH(-/-) and TPH(+/+). n.d.: below the minimal detection limit (< 5 pg/µl); *: statistically significant (p < 0,05), compared to all the other analysed mouse lines.
   (E) Trp, 5-HT and 5-HIAA in the hippocampus and the frontal cortex, two serotonergic projection areas of 129vJ, C57BL/6, TPH(-/-) and TPH(+/+)-mice and in the epiphysis of TPH(-/-)-mice and TPH(+/+)-mice. *: statistically significant (p < 0,05) compared to all other investigated mouse lines; +: statistically significant (p < 0,05) compared to TPH(+/+)-mice, but not to other laboratory mouse lines.
   (F) Immunohistochemical comparison of 5-HT in the duodenum of TPH(-/-)-mice (left) and wildtype mice (right). An anti-5-HT-staining is absent in the duodenum of the TPH(-/-)-mice, whereas enterochromaffin cells of TPH(+/+)-mice showed a strong staining.
   (G-I) Immunohistochemical comparison of 5-HT (E) and TPH (F) in the nuclei raphe of TPH(-/-)-mice (left) and wildtype mice (right).
   (G) Schematic presentation of the cut surface of the vibratome cut. Short additional picture: the vertical line shows the position in a side view of the brain. Large additional picture: Zoom of the stained areas, presented in the same way as in (H) and (I).
      4n: Nervus trochlearis or its root; DRD: dorsal nucleus raphe, dorsal part; DRV: dorsal nucleus raphe, ventral part; DRVL: dorsal nucleus raphe, ventrolateral part; mlf: medial longitudinal fascicle; MnR: medial nucleus raphe; PMnR: paramedial nucleus raphe; scp: Peduncu-lus cerebellaris superior (branchium conjunctivum).
   (H) anti-5-HT-staining of TPH(-/-)-mice and TPH(+/+)-mice.
   (I) anti-TPH-staining of TPH(-/-)-mice and TPH(+/+)-mice.
**Figure 2****:**
   Sequence and expression of mouse snTPH *(Mus musculus).*
   (A) The sequence comparison between the deduced amino acid sequences of snTPH and pTPH shows the high homology between the two enzymes, with the highest homology being in the catalytic region (aa 149 to 450).
   (B) Reversed phase-HPLC-FD chromatograms from the TPH activity assays. (above) non-transfected COS7-cells; (middle) COS7-cells, transiently transfected with a eukaryotic expression vector containing the mouse snTPH-cDNA; (below) P815 mouse mastocytoma cells with a high endogenous TPH-activity. 5-hydroxylated tryptophane (5-HT) was eluted with a residence time of 5 minutes.
   (C) RNase protection assay with specific probes for the two isoforms indicated the presence of pTPH mRNA in the duodenum, the thymus gland and the spleen of wildtype animals, but not of TPH(-/-)-mice. snTPH mRNA is exclusively present in the brain, independent of the genotype.
   (D) RNase protection assays specific for snTPH (lanes 1-3) and pTPH (lanes 4-6): lane 1: 30 µg brain-RNA, lane 2: 30 µg duodenum-RNA, lane 3: 30 µg P815 RNA, lane 4: 80 µg brain-RNA, lane 5: 80 µg duodenum-RNA, lane 6: 10 µg p815 RNA.
      P815-cells express very large amounts of pTPH-mRNA, but no detectable snTPH-mRNA.
   (E) The quantitative analysis of snTPH-mRNA in the brain-RNA of wildtype-animals and TPH(-/-)-mice, performed by using the RNase-protection assay, did not show an influence on pTPH-decrease. β-actin mRNA was used as an internal standard.
   (F) Comparison of pTPH-mRNA (left) and snTPH -mRNA (right) in the pons- and mid-brain-RNA of wildtype animals. Lane 1-3: 50 µg RNA for pTPH, lane 4-6: 30 µg RNA for snTPH. β-actin mRNA was used as an internal standard.

**Table 2: sequences of snTPHs described herein**

| |
|---|
| SEQ ID No.1: cDNA-sequence of the neuronal tryptophane hydroxylase (nTPH) of the human (Homo sapiens) |
| |
| |
| SEQ ID No. 2: Amino acid sequence of the neuronal tryptophane hydroxylase (nTPH) of the human (Homo sapiens) |
| |
| SEQ ID No. 3: cDNA-sequence of the neuronal tryptophane hydroxylase (nTPH) of the mouse (Mus musculus) |
| |
| |
| SEQ ID No. 4: Amino acid sequence of the neuronal tryptophane hydroxylase (nTPH) of the mouse (Mus musculus) |
| |
| |
| SEQ ID No. 5: cDNA-sequence of the neuronal tryptophane hydroxylase (nTPH) of the rat (Rattus norvegicus) |
| |
| |
| SEQ ID No. 6: Amino acid sequence of the neuronal tryptophane hydroxylase (nTPH) of the rat (Rattus norvegicus) |
| |

## Claims

1. An isolated nucleic acid sequence for use in medicine, encoding for a polypeptide having neuronal tryptophane hydroxylase activity, which is selected from the group of:
a) a nucleic acid sequence with the sequence depicted in SEQ ID No: 1 or SEQ ID No: 3 or SEQ ID No: 5, and
b) nucleic acid sequences encoding for polypeptides with the biological activity of a neuronal tryptophane hydroxylase which display more than 90 % homology to the amino acid sequences given in SEQ ID No: 2, SEQ ID No: 4 or SEQ ID No: 6.

2. A polypeptide for use in medicine, encoded by a nucleic acid sequence according to claim 1.

3. The polypeptide according to claim 2 for use in medicine, specified by the sequence of SEQ ID No: 2 or SEQ ID No: 4 or SEQ ID No: 6.

4. The isolated nucleic acid sequence according to claim 1 and the polypeptide according to claim 2 or 3, wherein the use in medicine is a use for detecting a predisposition, for diagnosis, for therapy or for monitoring of neurological diseases.

5. A recombinant nucleic acid molecule for use in medicine, containing a nucleic acid sequence according to claim 1, wherein the nucleic acid sequence is connected in an anti-sense or sense-direction with one or several regulatory signals.

6. A vector for use in medicine, containing a nucleic acid sequence according to claim 1 or a recombinant nucleic acid molecule according to claim 5.

7. A recombinant prokaryotic or eukaryotic non-human host organism for use in medicine containing at least one nucleic acid sequence according to claim 1 or at least one recombinant nucleic acid molecule according to claim 5 or at least one vector according to claim 6.

8. The recombinant prokaryotic or eukaryotic non-human host organism according to claim 7, wherein this organism is a microorganism or an animal.

9. Use of a polypeptide according to claim 2 or 3 as an antigen for the production of specific polyclonal or monoclonal antibodies or antibody mixtures for use in medicine directed against polypeptides according to claim 2 or 3.

10. Polyclonal or monoclonal antibody or antibody mixtures for use in medicine, which recognize specific polypeptides according to claim 2 or 3.

11. Use of a polypeptide according to claim 2 or 3 for identifying a specific inhibitor of the neuronal tryptophane hydroylase activity in vitro, whereby the polypeptide is expressed from a cDNA.

12. A method for isolating a compound that binds to the polypeptide according to claim 2 or 3, comprising:
(a) contacting a mammalian cell which expresses the polypeptide of claim 2 or 3 having a neuronal tryptophane hydroxylase activity with a compound; and
(b) detecting the presence of the compound which binds to the polypeptide of claim 2 or 3.

13. Use of a nucleic acid sequence according to claim 1, of a recombinant nucleic acid molecule according to claim 5 or of a polypeptide according to claim 2 or 3 in a Two-Hybrid-System for identifying/discovering proteins which have specific binding affinities for a polypeptide according to claim 2 or for identifying nucleic acids, which encode for proteins having specific binding affinities for a polypeptide according to claim 2 or 3.

14. A non-human transgenic animal for use in medicine containing a nucleic acid sequence selected from the group of:
a) a nucleic acid sequence with the sequence depicted in SEQ ID No: 1 or SEQ ID No: 3 or SEQ ID No: 5, and
b) nucleic acid sequences encoding for polypeptides with the biological activity of a neuronal tryptophane hydroxylase which display more than 90 % homology to the amino acid sequences given in SEQ ID No: 2, SEQ ID No: 4 or SEQ ID No: 6 in a non-functional form.

15. The transgenic animal according to claim 14, wherein the animal is a mouse, a rat, a sheep, a bovine animal, or a pig.

## Patentansprüche

1. Isolierte Nukleinsäuresequenz zur Verwendung in der Medizin, die für ein Polypeptid kodiert, das eine neuronale Tryptophanhydroxylaseaktivität aufweist, die ausgewählt ist aus der Gruppe von:
a) einer Nukleinsäuresequenz mit der in SEQ ID Nr: 1 oder SEQ ID Nr: 3 oder SEQ ID Nr: 5 dargestellten Sequenz, und
b) Nukleinsäuresequenzen, die für Polypeptide mit der biologischen Aktivität einer neuronalen Tryptophanhydroxylase kodieren, die mehr als 90 % Homologie zu den in SEQ ID Nr: 2, SEQ ID Nr: 4 oder SEQ ID No: 6 gegebenen Aminosäuresequenzen zeigen.

2. Polypeptid zur Verwendung in der Medizin, kodiert durch eine Nukleinsäuresequenz nach Anspruch 1.

3. Polypeptid nach Anspruch 2 zur Verwendung in der Medizin, angegeben durch die Sequenz von SEQ ID Nr: 2 oder SEQ ID Nr: 4 oder SEQ ID Nr: 6.

4. Isolierte Nukleinsäuresequenz nach Anspruch 1 und das Polypeptid nach Anspruch 2 oder 3, wobei die Verwendung in der Medizin eine Verwendung zum Nachweis einer Prädisposition, zur Diagnose, zur Therapie oder zur Überwachung von neurologischen Erkrankungen ist.

5. Rekombinantes Nukleinsäuremolekül zur Verwendung in der Medizin, enthaltend eine Nukleinsäuresequenz nach Anspruch 1, wobei die Nukleinsäuresequenz in einer anti-sense- oder sense-Richtung mit einem oder mehreren regulatorischen Signalen verbunden ist.

6. Vektor zur Verwendung in der Medizin, enthaltend eine Nukleinsäuresequenz nach Anspruch 1 oder ein rekombinantes Nukleinsäuremolekül nach Anspruch 5.

7. Rekombinanter prokaryontischer oder eukaryontischer nicht-menschlicher Wirtsorganismus zur Verwendung in der Medizin, enthaltend mindestens eine Nukleinsäuresequenz nach Anspruch 1 oder mindestens ein rekombinantes Nukleinsäuremolekül nach Anspruch 5 oder mindestens einen Vektor nach Anspruch 6.

8. Rekombinanter prokaryontischer oder eukaryontischer nicht-menschlicher Wirtsorganismus nach Anspruch 7, wobei der Organismus ein Mikroorganismus oder ein Tier ist.

9. Verwendung eines Polypeptids nach Anspruch 2 oder 3 als ein Antigen zur Herstellung von spezifischen polyklonalen oder monoklonalen Antikörpern oder Antikörpergemischen zur Verwendung in der Medizin, gerichtet gegen Polypeptide nach Anspruch 2 oder 3.

10. Polyklonaler oder monoklonaler Antikörper oder Antikörpergemische zur Verwendung in der Medizin, der/die spezifisch Polypeptide nach Anspruch 2 oder 3 erkennen.

11. Verwendung eines Polypeptids nach Anspruch 2 oder 3 zur Identifizierung eines spezifischen Inhibitors der neuronalen Tryptophanhydroxylaseaktivität *in vitro,* wobei das Polypeptid von einer cDNA exprimiert wird.

12. Verfahren zur Isolierung einer Verbindung, die an das Polypeptid nach Anspruch 2 oder 3 bindet, umfassend:
(a) in Kontakt bringen einer Säugerzelle, die das Polypeptid nach Anspruch 2 oder 3 exprimiert, das eine neuronale Tryptophanhydroxylaseaktivität aufweist, mit einer Verbindung; und
(b) Nachweisen der Anwesenheit der Verbindung, die an das Polypeptid nach Anspruch 2 oder 3 bindet.

13. Verwendung einer Nukleinsäuresequenz nach Anspruch 1, eines rekombinanten Nukleinsäuremoleküls nach Anspruch 5 oder eines Polypeptids nach Anspruch 2 oder 3 in einem Two-Hybrid-System zur Identifizierung/Auffindung von Proteinen, die spezifische Bindungsaffinitäten für ein Polypeptid nach Anspruch 2 aufweisen, oder zur Identifizierung von Nukleinsäuren die für Proteine kodieren, die spezifische Bindungsaffinitäten für ein Polypeptid nach Anspruch 2 oder 3 aufweisen.

14. Nicht-menschliches transgenes Tier zur Verwendung in der Medizin, enthaltend eine Nukleinsäuresequenz ausgewählt aus der Gruppe von:
a) einer Nukleinsäuresequenz mit der in SEQ ID Nr: 1 oder SEQ ID Nr: 3 oder SEQ ID Nr: 5 dargestellten Sequenz, und
b) Nukleinsäuresequenzen, die für Polypeptide mit der biologischen Aktivität einer neuronalen Tryptophanhydroxylase kodieren, die mehr als 90 % Homologie zu den in SEQ ID Nr: 2, SEQ ID Nr: 4 oder SEQ ID Nr: 6 angegebenen Aminosäuresequenzen zeigen,
in einer nicht-funktionellen Form.

15. Transgenes Tier nach Anspruch 14, wobei das Tier eine Maus, eine Ratte, ein Schaf, ein Horntier oder ein Schwein ist.

## Revendications

1. Séquence d'acides nucléiques isolée pour usage médical, codant pour un polypeptide présentant une activité de la tryptophane hydroxylase neuronale, sélectionnée à partir du groupe consistant en :
a) une séquence d'acides nucléiques avec la séquence représentée dans SEQ n° 1 ou SEQ ID n° 3 ou SEQ ID n° 5, et
b) des séquences d'acides nucléiques codant pour des polypeptides présentant l'activité biologique de la tryptophane hydroxylase neuronale qui ont plus de 90 % d'homologie avec les séquences d'acides aminés données dans SEQ ID n° 2, SEQ n° 4 ou SEQ ID n° 6.

2. Polypeptide pour usage médical, codé par une séquence d'acides nucléiques conforme à la revendication 1.

3. Polypeptide conforme à la revendication 2 pour usage médical, spécifié par la séquence SEQ ID n°2 ou SEQ ID n° 4 ou SEQ ID n° 6.

4. Séquence d'acides nucléiques isolée conforme à la revendication 1 et polypeptide conforme à la revendication 2 ou 3, dans lequel l'usage médical est un usage visant à détecter une prédisposition, à établir un diagnostic, à traiter ou à surveiller les affections neurologiques.

5. Molécule d'acides nucléiques recombinante pour usage médical, contenant une séquence d'acides nucléiques conforme à la revendication 1, dans laquelle la séquence d'acides nucléiques est connectée dans une direction sens ou antisens à un ou plusieurs signaux régulateurs.

6. Vecteur pour usage médical, contenant une séquence d'acides nucléiques conforme à la revendication 1 ou une molécule d'acides nucléiques recombinante conforme à la revendication 5.

7. Organisme hôte non humain procaryote ou eucaryote recombinant pour usage médical, contenant au moins une séquence d'acides nucléiques conforme à la revendication 1 ou au moins une molécule d'acides nucléiques recombinante conforme à la revendication 5 ou au moins un vecteur conforme à la revendication 6.

8. Organisme hôte non humain procaryote ou eucaryote recombinant conforme à la revendication 7, où cet organisme est un micro-organisme ou un animal.

9. Utilisation d'un polypeptide conforme à la revendication 2 ou 3 en tant qu'antigène pour la production d'anticorps ou de mélanges d'anticorps monoclonaux ou polyclonaux spécifiques pour usage médical dirigés contre les polypeptides conformes à la revendication 2 ou 3.

10. Anticorps ou mélanges d'anticorps monoclonaux ou polyclonaux pour usage médical, qui reconnaissent des polypeptides spécifiques conformes à la revendication 2 ou 3.

11. Utilisation d'un polypeptide conforme à la revendication 2 ou 3 pour l'identification d'un inhibiteur spécifique de l'activité de la tryptophane hydroxylase neuronale in vitro, où le polypeptide est exprimé à partir d'un ADNc.

12. Procédé pour l'isolement d'un composé qui se lie au polypeptide conforme à la revendication 2 ou 3, comprenant :
(a) la mise en contact d'une cellule de mammifère qui exprime le polypeptide de la revendication 2 ou 3 présentant une activité de la tryptophane hydroxylase neuronale avec un composé ; et
(b) la détection de la présence du composé qui se lie au polypeptide de la revendication 2 ou 3.

13. Utilisation d'une séquence d'acides nucléiques conforme à la revendication 1, d'une molécule d'acides nucléiques recombinante conforme à la revendication 5 ou d'un polypeptide conforme à la revendication 2 ou 3 dans un système double hybride pour l'identification/la découverte de protéines présentant des affinités de liaison spécifiques pour un polypeptide conforme à la revendication 2 ou pour l'identification d'acides nucléiques, qui codent pour des protéines présentant des affinités de liaison spécifiques pour un polypeptide conforme à la revendication 2 ou 3.

14. Animal transgénique non humain pour usage médical contenant une séquence d'acides nucléiques sélectionnée à partir du groupe consistant en :
a) une séquence d'acides nucléiques avec la séquence représentée dans SEQ ID n° 1 ou SEQ ID n° 3 ou SEQ ID n° 5, et
b) des séquences d'acides nucléiques codant pour des polypeptides présentant l'activité biologique de la tryptophane hydroxylase neuronale qui ont plus de 90 % d'homologie avec les séquences d'acides aminés données dans SEQ ID n° 2, SEQ ID n° 4 ou SEQ ID n° 6 dans une forme non fonctionnelle.

15. Animal transgénique conforme à la revendication 14, où l'animal est une souris, un rat, un mouton, un bovin ou un porc.
